(19) Europäisches Patentamt
European Patent Office
Office européen des brevets

(11) Veröffentlichungsnummer : **0 415 190 B1**

(12) **EUROPÄISCHE PATENTSCHRIFT**

(45) Veröffentlichungstag der Patentschrift : **19.01.94 Patentblatt 94/03**

(51) Int. Cl.$^5$ : **C07C 47/225,** C07C 33/05, C07D 311/96, A61K 7/46

(21) Anmeldenummer : **90115687.7**

(22) Anmeldetag : **16.08.90**

(54) **Cycloalkylidenderivate.**

(30) Priorität : **26.08.89 DE 3928242**

(43) Veröffentlichungstag der Anmeldung : **06.03.91 Patentblatt 91/10**

(45) Bekanntmachung des Hinweises auf die Patenterteilung : **19.01.94 Patentblatt 94/03**

(84) Benannte Vertragsstaaten : **CH DE ES FR GB LI NL**

(56) Entgegenhaltungen :
**DE-B- 2 006 388**
**DE-B- 2 065 172**
**DE-B- 2 718 549**
**DE-C- 962 074**
**GB-A- 1 438 100**
**US-A- 4 186 103**
**US-A- 4 192 782**
**US-A- 4 652 402**
**BULL. SOCIETE CHIMIQUE DE FRANCE, Paris, 1964 PIERRE CRESSON "Application de latransposition de Claisena la synthese d'aldehydes gamma, delta-ethyleniques" Seiten 2618-2628**

(73) Patentinhaber : **BASF Aktiengesellschaft**
**Carl-Bosch-Strasse 38**
**D-67063 Ludwigshafen (DE)**

(72) Erfinder : **Huellmann, Michael, Dr.**
**Siegfriedstrasse 41**
**D-6148 Heppenheim (DE)**
Erfinder : **Becker, Rainer, Dr.**
**Im Haseneck 22**
**D-6702 Bad Duerkheim (DE)**
Erfinder : **Janitschke, Lothar, Dr.**
**Wormser Gasse 9 a**
**D-6711 Kleinniedesheim (DE)**
Erfinder : **Lauterbach, Gerald, Dr.**
**Arminstrasse 38 a**
**D-6140 Bensheim (DE)**
Erfinder : **Barth, Wolfgang, Dr.**
**Boeddinghausstr.13**
**D-5600 Wuppertal 11 (DE)**
Erfinder : **Jansen, Klaas**
**Tannenstrasse 30**
**D-6700 Ludwigshaven (DE)**

EP 0 415 190 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Cycloalkylidenderivate der allgemeinen Formeln Ia bis Ic

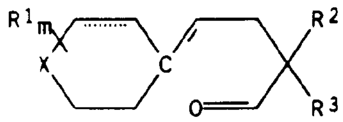

Ia

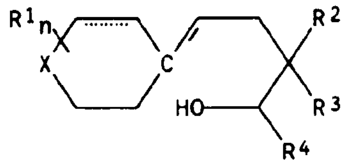

Ib

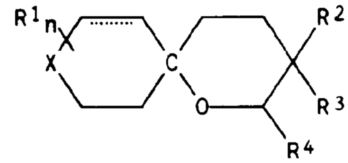

Ic

in denen die gestrichelte Linie für eine mögliche zusätzliche chemische Bindung steht und die Variablen die folgende Bedeutung haben:

- $R^1$ gleiche oder verschiedene $C_1$-$C_4$-Alkyl- oder $C_2$-$C_4$-Alkenylgruppen
- $R^2$, $R^3$ Methyl- oder Ethylgruppen
- $R^4$ Wasserstoff oder einen der Reste $R^1$
- X Sauerstoff, eine Methylengruppe oder eine chemische Bindung
- m 0 bis 3 oder, wenn X die Bedeutung einer Methylengruppe hat, 1 bis 3 oder, wenn X eine Methylengruppe und $R^1$ eine Methylgruppe bedeuten, 2 bis 3
- n 0 bis 3.

Außerdem betrifft die Erfindung Verfahren zur Herstellung der Cycloalkylidenderivate Ia bis Ic sowie deren Verwendung als Duftstoffe in kosmetischen Präparaten und Pflegemitteln für Haushalt und Industrie.

Aus der Arbeit von Cresson (Bull. Soc. Chim. France (1964), 10, 2618-2628) sind Cycloalkylidenderivate vom Typ Ia mit der Struktureinheit eines unsubstituierten Cyclohexylrings bekannt. Die Verbindung, in der $R^2$ und $R^3$ für Methylgruppen steht, weist, wie festgestellt wurde, eine frische, grüne, krautige Duftnote auf.

Ferner ist aus DE-A-96 20 74 die Herstellung von 4-[2',6',6'-Trimethyl-cyclohexyliden]-2-methyl-2-buten-1-al und 4-[2',6',6'-Trimethyl-2'-cyclohexenyliden]-2-methyl-2-buten-1-al bekannt. Beide Verbindungen werden beschrieben als wertvolle Zwischenprodukte für die Synthese von Vitamin A und von Carotinoiden sowie als Riechstoffe mit fixierenden Eigenschaften, die an β-Ionon erinnern.

Spirocyclische Ether, mit der Grundstruktur Ic sind als Duftstoffe aus den US-A 4 010 286, 4 186 103, 4 192 782 und 4 240 447 bekannt, in denen im Unterschied zu Ic $R^2$ und $R^3$ für Wasserstoff stehen und die in 4-Stellung des Dehydropyranringes eine Methyl- oder Methylengruppe tragen.

Der Erfindung lagen neue Verbindungen mit neuartigen Dufteigenschaften als Aufgabe zugrunde.

Demgemäß wurden die eingangs definierten Verbindungen Ia bis Ic gefunden. Weiterhin wurden Verfahren zu deren Herstellung, deren Verwendung als Duftstoffe, sowie sie enthaltende Zubereitungen von Riechstoffkompositionen, gefunden.

Unter den Verbindungen Ia sind diejenigen bevorzugt, in denen X eine chemische Bindung oder eine Methylengruppe bedeutet und $R^1$ für Alkylsubstituenten in 2- und 4-Position steht.

Beispiele für derartige Verbindungen sind:

4-Cyclopentyliden-2,2-dimethylbutanal

4-[2,4,4'-Trimethyl-cyclopentyliden]-2,2-dimethyl-butanal

4-[4-Isopropyl-cyclohexyliden]-2,2-dimethylbutanal.

Man erhält die Verbindungen Ia, indem man ein cyclisches Keton (II) mit einem Vinylmagnesiumhalogenid in das Vinylcarbinol (III) überführt, III mit Phosgen oder Thionylchlorid in an sich bekannter Weise zu IV umsetzt und dieses mit einem Aldehyd V reagieren läßt:

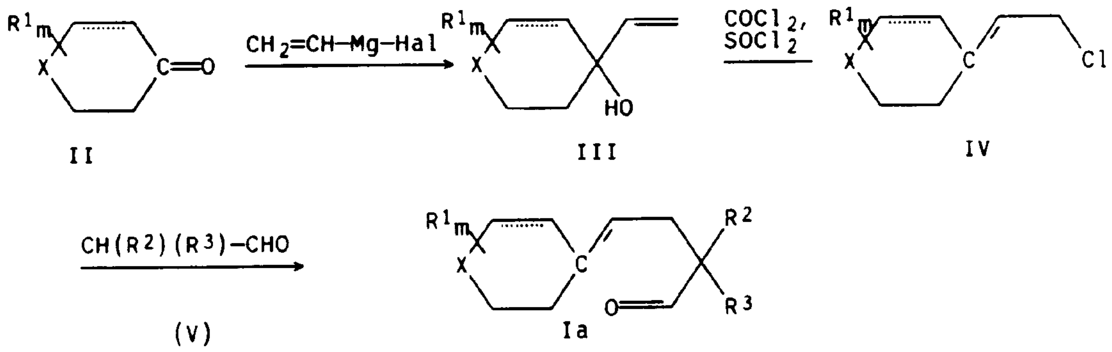


Die Umsetzung von cyclischen Ketonen mit einem Vinylmagnesiumhalogenid ist an sich bekannt (Houben-Weyl, "Methoden der organischen Chemie", Band XIII/2a (1973), S. 47-527), so daß sich detaillierte Angaben hierüber erübrigen.

Die Vinylcarbinole III können durch Umsetzung mit einem Chlorierungsreagens, vorzugsweise Phosgen oder Thionylchlorid, bei (-80) bis 150, vorzugsweise (-20) bis 20°C in die Verbindungen IV überführt werden. Die Umsetzungen kann man löungsmittelfrei oder in einem Lösungsmittel durchführen. Geeignete Lösungsmittel sind Ether wie Diethylether, Ester wie Essigsäureethylester, aliphatische und aromatische Kohlenwasserstoffe wie Hexan oder Toluol, Amide wie Dimethylformamid, Sulfoxide wie Dimethylsulfoxid oder Mischungen dieser Lösungsmittel.

Schließlich können die Allylchloride (IV) zur Darstellung der Cycloalkylidenalkanale mit einem 1- bis 2-fachen Überschuß, vorzugsweise 1- bis 1,5-fachen Überschuß an Aldehyd V zur Reaktion gebracht werden. Die Reaktion kann man zweckmäßigerweise im basischem Milieu, vorzugsweise in einem Zweiphasensystem aus einer wäßrigen Alkalimetallhydroxidlösung und Toluol unter Zuhilfenahme eines Phasentransferkatalysators durchführen. Als Phasentransferkatalysatoren kommen quartäre Ammoniumsalze wie Tetrabutylammoniumiodid in Betracht.

Bevorzugte Verbindungen Ib sind solche mit einem Cyclohexylrest, der entweder keinen, gleiche oder verschiedene Reste $R^1$ in den Positionen 2, 3, 4 oder 6 trägt. Beispiele für derartige Verbindungen sind:
4-[Cyclohexyliden]-2,2-dimethylbutanol,
4-[3,3-Dimethyl-cyclohexyliden]-2,2-dimethylbutanol,
5-[2,5,6-Trimethyl-cyclohex-2-enyliden]-3,3-dimethyl-pentan-2-ol,
4-[4-Isopropyl-cyclohexyliden]-2,2-dimethylbutanol,
5-[4-Isopropyl-cyclohexyliden]-3,3-dimethyl-pentan-2-ol.

Die Verbindungen Ib kann man durch die Reduktion der Formyldgruppe der Cycloalkylidenalkanale Ia, vorzugsweise mit komplexen Metallhydriden wie z.B. Lithiumaluminiumhydrid oder Natriumborhydrid, bzw. durch die Umsetzung mit Alkyl- oder Vinylmagnesiumhalogeniden, erhalten.

Die Reduktion mit den Metallhydriden kann in Alkoholen wie Methanol, Ethanol oder tert.-Butanol bei 0 bis 50°C, vorzugsweise bei 25°C durchgeführt werden. Die Umsetzung der Cycloalkylidenalkanale Ia mit den Grignardverbindungen erfolgt nach allgemein literaturbekannten Methoden, weshalb nähere Angaben hierzu nicht erforderlich sind (Houben-Weyl, "Methoden der Organischen Chemie", Band XIII/2a (1973), S. 47-527).

Diejenigen spirocyclischen Ether (Ic), die aus zwei sechsgliedrigen Ringen aufgebaut sind und die in 7- oder 8-Position einen der Reste $R^1$ tragen, zeigen die interessantesten Duftstoffnoten. Beispiele für solche Verbindungen sind:
3,3'-Dimethyl-8-isopropyl-1-oxaspiro[5,5]undecan
3,3'-Dimethyl-7,7'-dimethyl-1-oxaspiro[5,5]undecan
2-Methyl-3,3'-dimethyl-7,7'-dimethyl-1-oxaspiro[5,5]undecan
2-Vinyl-3,3'-dimethyl-7,7'-dimethyl-1-oxaspiro[5,5]undecan
2-Methyl-3,3'-dimethyl-7,7'-dimethyl-1-oxaspiro[5,5]undecan.

Die Verbindungen Ic erhält man vorzugsweise durch die Cyclisierung der entsprechenden Cycloalkylidenalkanole Ib in Gegenwart saurer Katalysatoren.

Die Reaktion kann man bei einer Temperatur zwischen (-80) und 100, vorzugsweise zwischen (-20) und 50°C durchführen. Die Umsetzung kann lösungsmittelfrei oder in einem inerten Lösungsmittel durchgeführt werden. Geeignete Lösungsmittel sind Kohlenwasserstoffe wie Toluol, chlorierte Kohlenwasserstoffe wie Methylenchlorid und Chlorbenzol und Ether wie Tetrahydrofuran und Methyl-tert.-butylether. Als saure Katalysatoren kommen anorganische und organische Säuren wie Schwefelsäure, Phosphorsäure, Salzsäure, Essigsäure, Oxalsäure, p-Toluolsulfonsäure, saure Ionenaustauscher sowie Lewis-Säuren wie Zinkchlorid und Bortrifluorid in Betracht.

Die erfindungsgemäßen Verbindungen Ia und Ib weisen frische und blumige Duftnoten auf, wogegen die spirocyclischen Ether einen ausgeprägt animalischen Duftcharakter haben. Sie dienen für sich allein oder vorzugsweise mit anderen Duftstoffen in den hierfür üblichen Kombinationen zur Herstellung von Parfümen, zur Parfümierung, das heißt, zur Verleihung, Verbesserung oder Modifizierung der Dufteigenschaften von kosmetischen Präparaten sowie von Waschmitteln, Spülmitteln und Pflegemitteln für den Haushalt und den industriellen Gebrauch.

Beispiele 1 bis 6

Herstellung der Verbindungen Ia

3,5 mol eines cyclischen Ketons (II) wurden bei 25°C innerhalb von 2 Stunden mit einer Lösung aus 4,5 mol Vinylmagnesiumchlorid in 3 l Tetrahydrofuran versetzt. Nach 12-stündigem Nachrühren wurde die Grignardverbindung mit 450 ml Wasser hydrolisiert, wonach das Gemisch mit Methyl-tert.-butylether extrahiert wurde. Die übliche Aufarbeitung lieferte das Vinylcarbinol III.

Zu 2,0 mol des Vinylcarbinols (III), gelöst in einer Mischung aus 600 ml Toluol und 180 ml Dimethylformamid, wurden bei 0°C innerhalb von 4 Stunden 2,2 mol Phosgen eingeleitet. Anschließend wurde die Reaktionsmischung noch 1 Stunde nachgerührt. Zur Aufarbeitung wurde das Gemisch zweimal mit je 1 l Wasser ausgewaschen, mit $NaHCO_3$-Lösung neutralisiert, und die organische Phase eingeengt. Diese Lösung wurde unmittelbar in der Folgereaktion eingesetzt.

Zu einer Lösung von 27 mmol Tetrabutylammoniumiodid in 25 %iger Natronlauge wurden bei 50°C 2,2 mol Isobutyraldehyd (V) und 1,65 mol Allylchlorid IV, gelöst in 600 ml Toluol/Dimethylformamid, zugetropft. Anschließend wurde 16 Stunden bei 50°C und 4 Stunden bei 70°C nachgerührt. Zur Aufarbeitung wurde die organische Phase abgetrennt, mit kaltem Wasser gewaschen, mit Magnesiumsulfat getrocknet und nach Einengen über eine Vigreuxkolonne destilliert.

Die Einzelheiten dieser Versuche sowie deren Ergebnisse sind Tabelle 1 zu entnehmen.

Tabelle 1 Herstellung der Verbindungen Ia

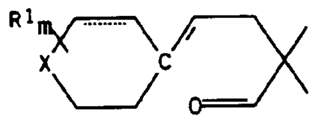

| | X | $R^1$ | | III Ausb. Sdp. | IV Ausb. Sdp. | Ia Ausb. Sdp. | Duftnote |
|---|---|---|---|---|---|---|---|
| 1 | chem. Bindung | H | Einfachbindung | 65 % 36°/0,53 mbar (0,4 mm) | 58 % Rohprodukt | 53 % 60°/0,53 mbar (0,4 mm) | fruchtig |
| 2 | $CH_2$ | 2,3,6-$CH_3$ | Einfachbindung | 57 % 64°/0,53 mbar (0,4 mm) | 46 % 60°/0,2 mm | 46 % 136°C/0,6 mbar (0,45 mm) | brenzlig würzig |
| 3 | $CH_2$ | 4-$CH(CH_3)_2$ | Einfachbindung | 77 % 123°/50,65 mbar (38 mm) | 82 % Rohprodukt | 56 % 76°/0,4 mbar (0,3 mm) | blumig |
| 4 | $CH_2$ | 4-$CH(CH_3)_2$ | Doppelbindung | 90 % 73°/0,67 mbar (0,5 mm) | 43 % 85°/0,4 mm | 51 % 142°/0,53 mbar (0,4 mm) | blumig |
| 5 | $CH_2$ | 3,3-$CH_3$ | Einfachbindung | 73 % 60°/0,53 mbar (0,4 mm) | 55 % 53°C/0,4 mbar (0,3 mm) | - | - |
| 6 | - | 2,4,4-$CH_3$ | Einfachbindung | - | 25 % Rohprodukt | 56 % 95°/0,2 mbar (0,15 mm) | Rose grünlich |

1 Die Rohprodukte wurden ohne Reinigung als Toluollösung weiter umgesetzt.

Beispiel 7 bis 14

1 mol Cyclohexylidenalkanal (Ia) wurden bei 25°C innerhalb von 2 Stunden zu einer Lösung von 0,63 mol Natriumborhydrid in 1,2 l Ethanol zugetropft. Anschließend wurde die Reaktionsmischung noch 12 Stunden bei 25°C gerührt. Zur Aufarbeitung wurde die Reaktionslösung eingeengt, der Rückstand in Methyl-tert.-butylether aufgenommen und mit 10 %iger $H_2SO_4$ versetzt. Die organische Phase wurde abgetrennt, mit Natriumhydrogencarbonat-Lösung neutralisiert, über Magnesiumsulfat getrocknet, eingeengt und destilliert.

Die in der nachstehenden Tabelle aufgeführten Alkohole wurden entweder analog oder durch Umsetzung mit einer entsprechenden Grignardverbindung hergestellt.

Tabelle 2 Darstellung der Verbindungen Ib

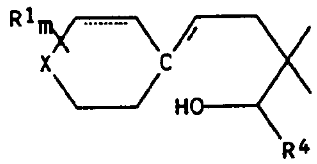

| | X | $R^1$ | $R^4$ | Ausb. | Sdp. | Duftnote |
|---|---|---|---|---|---|---|
| 7 | $CH_2$ | - | H | 63 % | 79°C/ 0,27 mbar (0,2 mm) | fruchtig, rose |
| 8 | - | - | H | 76 % | 70°C/0,2 mbar (0,15 mm) | fruchtig |
| 9 | $CH_2$ | 2,3,6-$CH_3$ | H | 79 % | 111°C/0,2 mbar (0,15 mm) | metallisch, chemisch |
| 10 | - | 2,4,4'-$CH_3$ | H | 68 % | 81°C/0,27 mbar (0,2 mm) | holzig, fäkalisch |
| 11 | - | 2,4,4'-$CH_3$ | $CH_3$ | 74 % | 94°C/0,24 mbar (0,18 mm) | aldehydig, grün |
| 12 | - | - | $CH_3$ | 75 % | 82°C/0,24 mbar (0,18 mm) | fettig, blumig |
| 13 | $CH_2$ | 4-$CH(CH_3)_2$ | H | 76 % | 100°C/0,4 mbar (0,3 mm) | fruchtig, blumig |
| 14 | $CH_2$ | 4-$CH(CH_3)_2$ | $CH_3$ | 67 % | 120°C/0,4 mbar (0,3 mm) | holzig, würzig |

Beispiel 15 bis 19

7,5 ml Bortrifluorid-etherat in 45 ml Methylenchlorid wurden bei 0°C mit 60 mmol Cyclohexylidenalkanol (Ib) tropfenweise versetzt. Nach 2-stündigem Nachrühren wurde die Reaktionslösung auf Eiswasser gegeben und anschließend mit Methylenchlorid extrahiert. Die übliche Aufarbeitung lieferte die spirocyclischen Ether Ic.

Die Einzelheiten dieser Versuche sowie deren Ergebnisse sind Tabelle 3 zu entnehmen.

Tabelle 3 Herstellung der Verbindungen Ic

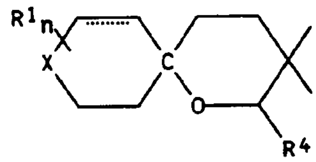

| | X | $R^1$ | $R^4$ | Ausb. | Duftnote 100 % | 10 %[1] |
|---|---|---|---|---|---|---|
| 15 | $CH_2$ | - | H | 75 % | - | - |
| 16 | $CH_2$ | 7,7-$CH_3$ | H | 80 % | fäkalisch, lederartig | lederartig |
| 17 | $CH_2$ | 7,7-$CH_3$ | $CH_3$ | 80 % | fäkalisch, krautig | krautig |
| 18 | $CH_2$ | 7,7-$CH_3$ | $-CH=CH_2$ | 78 % | fäkalisch, Salbei | Salbei |
| 19 | $CH_2$ | 8-$CH(CH_3)_2$ | H | 75 % | Zibet | Zibet |

1) 10 %ige Lösung in Ethylalkohol

## Patentansprüche

**1.** Cycloalkylidenderivate der allgemeinen Formeln Ia bis Ic

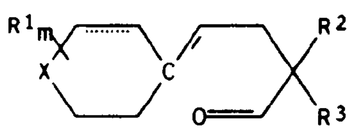

Ia

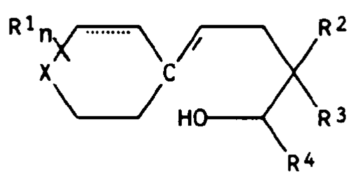

Ib

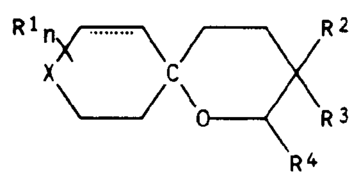

Ic

in denen die gestrichelte Linie für eine mögliche zusätzliche chemische Bindung steht und die Variablen die folgende Bedeutung haben:

| | |
|---|---|
| $R^1$ | gleiche oder verschiedene $C_1$-$C_4$-Alkyl- oder $C_2$-$C_4$-Alkenylgruppen |
| $R^2$, $R^3$ | Methyl- oder Ethylgruppen |
| $R^4$ | Wasserstoff oder einen der Reste $R^1$ |
| X | Sauerstoff, eine Methylengruppe oder eine chemische Bindung |
| m | 0 bis 3 oder, wenn X die Bedeutung einer Methylengruppe hat, 1 bis 3 oder, wenn X eine Methylengruppe und $R^1$ eine Methylgruppe bedeuten, 2 bis 3 |
| n | 0 bis 3. |

**2.** Verfahren zur Herstellung von Cycloalkylidenalkanalen der allgemeinen Formel Ia, dadurch gekennzeichnet, daß man ein cyclisches Keton (II)

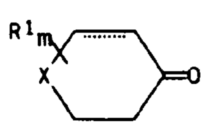

II

in an sich üblicher Weise mit einem Vinylmagnesiumhalogenid umsetzt, die erhaltenen Vinylcarbinole (III)

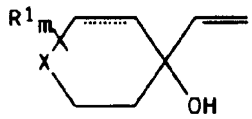

III

in an sich bekannter Weise mit Phosgen oder Thionylchlorid in die analogen Allylchloride (IV)

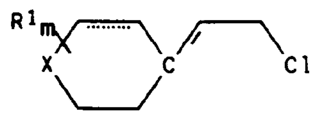

IV

überführt und diese anschließend mit einem Aldehyd (V)

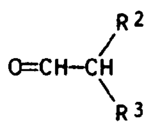

V

in Gegenwart einer Base zu den korrespondierenden Cycloalkylidenalkanalen Ia umsetzt.

3. Verfahren zur Herstellung der Cycloalkylidenalkanole Ib, dadurch gekennzeichnet, daß man die Formylgruppe von Ia oder eine Verbindung vom Typ Ia, in der m die Bedeutung von n hat, hydriert oder mit einer Grignardverbindung $R^4$-Mg-Hal umsetzt, wobei Hal für Chlor, Brom oder Iod steht.

4. Verfahren zur Herstellung von spirocyclischen Ethern der allgemeinen Formel Ic gemäß Anspruch 1, dadurch gekennzeichnet, daß man die entsprechenden Cycloalkylidenalkanole Ib in Gegenwart von sauren Katalysatoren cyclisiert.

5. Verwendung der Cycloalkylidenderivate gemäß Anspruch 1 zur Verleihung, Verbesserung oder Modifizierung der Dufteigenschaften von kosmetischen Präparaten, Waschmitteln, Reinigungsmitteln, Haushaltspflegemitteln und ähnlichen Zubereitungen.

6. Verwendung der Cycloalkylidenderivate gemäß Anspruch 1 für Duftstoffkompositionen.

## Claims

1. A cycloalkylidene derivative of the formula Ia, Ib or Ic

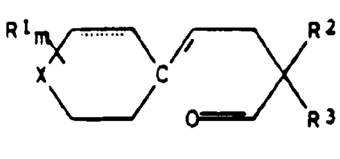

Ia

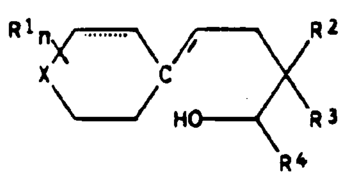

Ib

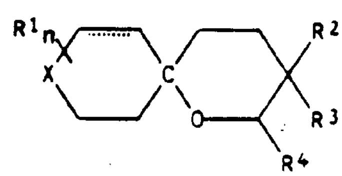

Ic

where the dashed line is a possible additional chemical bond, the radicals $R^1$ are identical or different $C_1$-$C_4$-alkyl or $C_2$-$C_4$-alkenyl groups, $R^2$ and $R^3$ are each methyl or ethyl, $R^4$ is hydrogen or one of the radicals $R^1$, X is oxygen, methylene or a chemical bond, m is from 0 to 3 or, when X is methylene, is from 1 to 3, or, when X is methylene and $R^1$ is methyl, is from 2 to 3 and n is from 0 to 3.

2. A process for the preparation of a cycloalkylidenealkanal of the formula Ia, wherein a cyclic ketone (II)

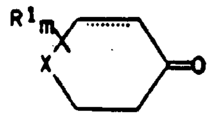

II

is reacted in a conventional manner with a vinylmagnesium halide, the resulting vinylcarbinol (III)

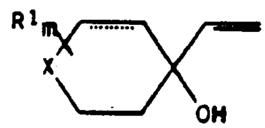

III

is converted in a conventional manner with phosgene or thionyl chloride into the analogous allyl chloride (IV)

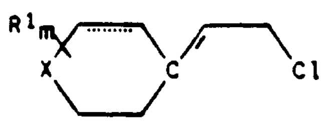

IV

and the latter is then reacted with an aldehyde (V)

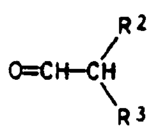

V

in the presence of a base to give the corresponding cycloalkylidenealkanal Ia.

3. A process for the preparation of a cycloalkylidenealkanol Ib, wherein the formyl group of Ia or a compound of the type Ia where m has the same meaning as n is hydrogenated or is reacted with a Grignard compound $R^4$-Mg-Hal, where Hal is chlorine, bromine or iodine.

4. A process for the preparation of a spirocyclic ether of the formula Ic as claimed in claim 1, wherein the corresponding cycloalkylidenealkanol Ib is cyclized in the presence of an acidic catalyst.

5. Use of a cycloalkylidene derivative as claimed in claim 1 for imparting fragrance properties to, or improving or modifying the fragrance properties of, cosmetic preparations, detergents, cleaning agents, household care agents and similar formulations.

6. Use of a cycloalkylidene derivative as claimed in claim 1 for fragrance compositions.

## Revendications

1. Dérivés de cycloalkylidène de formules générales Ia à Ic

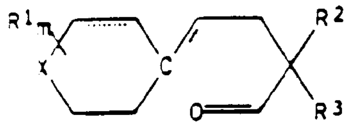 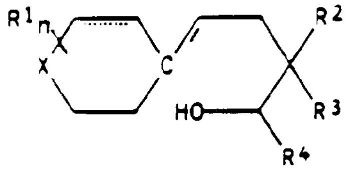 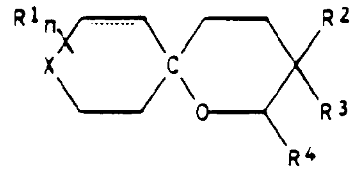

Ia Ib Ic

dans lesquelles la ligne en pointillés représente une liaison chimique supplémentaire éventuelle et les variables ont la signification suivante :

| | |
|---|---|
| $R^1$ | groupements alkyle en $C_1$-$C_4$ ou alcényle en $C_2$-$C_4$ identiques ou différents |
| $R^2$, $R^3$ | groupements méthyle ou éthyle |
| $R^4$ | hydrogène ou l'un des restes $R^1$ |
| X | oxygène, un groupement méthylène ou une liaison chimique |
| m | 0 à 3 ou, quand X a la signification d'un groupement méthylène 1 à 3 ou, quand X représente un groupement méthylène et $R^1$ un groupement méthyle, 2 à 3 |
| n | 0 à 3. |

2. Procédé de préparation de cycloalkylidène-alcanals de formule générale Ia, caractérisé en ce qu'on fait réagir une cétone cyclique (II)

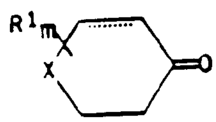

II

d'une manière connue en soi avec un halogénure de vinylmagnésium, on transforme le vinylcarbinol (III) obtenu

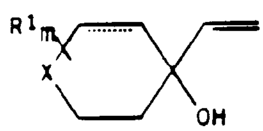

III

d'une manière connue en soi avec du phosgène ou du chlorure de thionyle en le chlorure allylique analogue (IV)

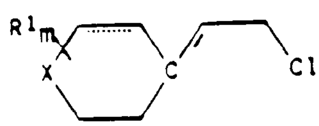

IV

et on fait ensuite réagir celui-ci avec un aldéhyde (V)

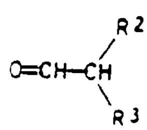

V

en présence d'une base pour obtenir le cycloalkylidène-alcanal correspondant Ia.

3. Procédé de préparation des cycloalkylidène-alcanols Ib, caractérisé en ce qu'on hydrogène ou on fait réagir avec un dérivé de Grignard $R^4$-Mg-Hal, où Hal représente le chlore, le brome ou l'iode, le groupement

formyle de Ia ou un composé du type Ia, où m a la signification de n.

4. Procédé de préparation d'éthers spirocycliques de formule générale Ic selon la revendication 1, caractérisé en ce qu'on cyclise les cycloalkylidène-alcanols correspondants Ib en présence de catalyseurs acides.

5. Utilisation des dérivés de cycloalkylidène selon la revendication 1 pour conférer un parfum ou améliorer ou modifier le parfum de préparations cosmétiques, de produits de lavage, de produits de nettoyage, de produits d'entretien ménagers et de préparations similaires.

6. Utilisation des dérivés de cycloalkylidène selon la revendication 1 pour des compositions de parfums.